# EUROPEAN PATENT APPLICATION

(11) **EP 1 790 333 A1**
(43) Date of publication of application: **30.05.2007**
(21) Application number: 06076841.3
(22) Date of filing: 05.10.2006
(51) Int. Cl.: A61K 9/28, A61K 31/19

(54) **Gastroresistant tablet comprising a butyric acid compound**

(30) Priority: 21.10.2005 IT MI20052000
(71) Applicant: Promefarm s.r.l., 20129 Milano (IT)
(72) Inventor: Zanarotti, Alessandro, 20129 Milano (IT)
(74) Representative: Marchi, Massimo

(57) **Abstract**

Gastroresistant tablet having (i) a core comprising butyric acid, or a salt or an ester thereof as an active ingredient, and at least one pharmaceutically acceptable excipient, (ii) an intermediate coating, and (iii) an outer gastroresistant coating, wherein the said intermediate coating comprises a cellulose compound and the said gastroresistant coating remains unchanged at an acidic pH and dissolves at pH ≥ 7.2.

## Description

This invention relates to a gastroresistant tablet comprising a butyric acid compound.

Butyric acid is a short chain fatty acid (SCFA), produced in the colon by autochthonous flora during the process of the fermentation of fibres present in foods of plant origin (fruit and vegetables), which reach the large intestine undigested.

Some properties of butyric acid are known, including the essential property of supporting the integrity of the colonic mucosa, for which it is the main source of energy through the activity of colonocytes (Velasquez O.C. et al., Butyrate and the colonocyte: Production, Absorption, Metabolism and Therapeutic Implications, in "Dietary Fibre in Health and Disease" edited by Kritchesvskj and Bonfield, Plenum Press, New York, 1977, pages 123-134).

Butyric acid is absorbed by colonocytes through the mechanisms of both passive diffusion and active transport, associated with the exchange of various ions - the absorption of sodium, chlorine and calcium on the one hand, and the secretion of potassium on the other.

The importance of butyric acid and other SCFA for the absorption of some electrolytes in the large intestine lies in its physiological action, an imbalance which may give rise to certain types of diarrhoea (Holtug K. et al., The colon in carbohydrate malabsorption: short-chain fatty acid, pH and osmotic diarrhoea, Scand. J. Gastroenterol. 1992, 27:545-552).

In addition to this SCFA are capable of stimulating the action of bifidobacteria in the large intestine and this can have some beneficial effects in improving lipid metabolism and increasing the bioavailability of some minerals (Glizard, D. et al., Non-digestible oligosaccharides used as prebiotic agent: mode of production and beneficial effect on animal and human health, Reprod. Nutr. Dev. 1999, 39: 563-588).

Butyric acid deficiency in the colon, either because of the absence of suitable bacterial flora or through an insufficient input of dietary fibre, may interfere with the physiological activity of the colonocytes, which draw upon butyric acid and SCFA as the essential fuel for their physiological functions.

In this respect, a supplemental input of butyric acid into the colon can be regarded as a dietary supplement capable of improving the natural recovery of the physiological functions of the cells in the colonic mucosa when changes occur in the fermentation process due to imbalances in the bacterial flora or an insufficient input of dietary fibre.

In addition to this, the use of butyric acid in some intestinal diseases has also been recommended in the literature.

However, because of its rapid metabolism and short plasmatic half-life the potential use of butyric acid both as a dietary supplement and as a drug has hitherto been restricted owing to the difficulty of achieving effective concentrations in the colon.

In addition to this, butyrates have an unpleasant odour, so that the patients reject it.

In order to overcome this problem US patent A-5 569 680 proposes the administration of glycerol tributyrate, which acts as a pro-drug for butyric acid in that is capable of releasing three units of butyric acid for each glycerol tributyrate molecule. However this administration is performed by enema, which is a very unsuitable and inconvenient form of administration for individuals engaged in an active life.

The need for an oral pharmaceutical form capable of transporting an effective concentration of butyric acid or a salt or an ester thereof into the colon is therefore still very much felt.

After many attempts the inventors have developed a tablet which contains butyric acid, or a salt or an ester thereof, as an active ingredient, and has the property of remaining unchanged in gastric environment (pH ≈ 1) and of disaggregating only after it has reached the colon region (pH ≥ 7.2), thus allowing a targeted release of the active ingredient at the intended site of action.

In particular, this invention relates to a gastroresistant tablet having (i) a core comprising butyric acid, or a salt or an ester thereof as an active ingredient, and at least one pharmaceutically acceptable excipient, (ii) an intermediate coating, and (iii) an outer gastroresistant coating, wherein the said intermediate coating comprises a cellulose compound and the said gastroresistant coating remains unchanged at an acidic pH and dissolves at pH ≥ 7.2.

A typical example of a salt is sodium butyrate. Typical examples of esters are methyl butyrate and triglyceryl tributyrate. In a preferred embodiment the aforesaid core comprises sodium butyrate.

Advantageously the dose of sodium butyrate is of 1000 mg/tablet.

Typically the core of the pharmaceutical composition according to this invention is obtained by pressing a granulate which comprises the active ingredient together with conventional pharmaceutical excipients. Advantageously these excipients are: diluting agents such as, for example, microcrystalline cellulose and anhydrous dibasic calcium phosphate, disaggregating agents such as, for example, cross-linked sodium carboxymethyl cellulose and crospovidone, anti-adhesion agents such as, for example talc, flowing agents such as, for example, colloidal silica, and lubricating agents such as, for example, magnesium stearate.

The compression of this granulate results in a core which has the mechanical properties required during the coating step(s).

A drawback discovered in preparation of the oral composition according to this invention is that sodium butyrate proves to be incompatible with known gastroresistant coatings because its alkalinity impairs the resistance of the gastroresistant coating to acids, so that the tablet breaks up at acid pH.

The inventors have found that this drawback can be overcome by providing the said intermediate coating comprising a cellulose compound.

Typical examples of suitable cellulose compounds are: hydroxypropylmethyl cellulose, methyl cellulose, ethylmethyl cellulose, hydroxymethyl cellulose, and mixtures thereof.

In addition to the cellulose compound the said intermediate coating may further contain other ingredients such as for example microcrystalline cellulose, stearic acid, colloidal anhydrous silica, talc, PEG and the like.

The gastroresistant coating according to this invention has the property of enabling the tablet to pass substantially unchanged throughout the gastrointestinal tract up to its terminal part, that is the colon, whereas when it reaches the colon the gastroresistant tablet according to this invention readily disaggregates, thus realising the active ingredient at its site of action.

Advantageously the gastroresistant coating according to this invention comprises shellac as an agent controlling release. Preferably it further comprises plasticizing agents such as for example triethyl citrate, base-forming agents such as for example ammonium carbonate, anti-adhesion agents such as for example talc, colouring agents such as for example titanium dioxide and yellow iron oxide, and flavouring agents such as for example vanillin and the like.

The following examples will illustrate this invention without however limiting it in any way.

**EXAMPLE 1**

| No. | Ingredient | Dose per tablet (mg) |
|---|---|---|
| Core | | |
| 1. | Sodium butyrate | 1000.00 |
| 2. | Microcrystalline cellulose | 200.00 |
| 3. | Colloidal silica | 7.00 |
| 4. | Anhydrous dibasic calcium phosphate | 105.00 |
| 5. | Cross-linked sodium carboxymethyl cellulose | 40.00 |
| 6. | Polyvinyl pyrrolidone CL | 33.00 |
| 7. | Talc | 20.00 |
| 8. | Magnesium stearate | 15.00 |

| Intermediate coating | | |
|---|---|---|
| 9. | Hydroxypropylmethyl cellulose | 32.00 |
| 10. | Microcrystalline cellulose | 4.00 |
| 11. | Stearic acid | 4.00 |

| Gastroresistant coating | | |
|---|---|---|
| 12. | Shellac | 13.00 |
| 13. | Triethyl citrate | 1.95 |
| 14. | Ammonium carbonate | 1.00 |
| 15. | Talc | 7.62 |
| 16. | Titanium dioxide | 0.87 |
| 17. | Yellow iron oxide | 0.13 |
| 18. | Vanillin | 0.13 |

| | | |
|---|---|---|
| a) ingredients from 1 to 8 were sieved with a square mesh having openings of 1.96 mm²; b) afterwards they were dry blended for 20 minutes at 50 r.p.m.; c) the thus obtained mixture was pressed in a rotary tablet machine equipped with 21 x 9.8 mm oval punches; d) the tablets were ground in an oscillating arm granulator with a mesh having square openings of 4 mm² and subsequently with the mesh having square openings of 1.69 mm²; e) the granulate was transferred to a blender and was mixed for 5 minutes; f) the mixture was pressed to the theoretical weight of 1420 mg per core using 21 x 9.8 mm oval punches; g) separately the ingredients from 9 to 11 (the mixture of ingredients is commercially available under the name of Sepifilm^{™} LP010) were poured under stirring into purified water in a dissolver and stirring was maintained. After approximately 1 hour the solution was filtered on a mesh having openings of 0.0225 mm² (N.B.: stirring of this coating solution was maintained throughout the film-coating operation); h) the cores from step f) were coated with the solution from step g) by spraying in a coating pad according to following parameters: tablet temperature: 45 - 49°C, bowl rotation: 6-7 r.p.m., spray pressure: 1.7 bar, total spraying time: 4 hours, mean weight of coated cores: 1460 mg; i) ingredient 14 was poured into purified water with stirring in a dissolver; j) after dissolution of ingredient 14 was complete, ingredient 12 was added, again with stirring. The mixture was heated to 50°C - 60°C and left at this temperature, under stirring, until complete dissolution occurred; k) the thus obtained solution was cooled to ambient temperature and ingredient 13 was poured into it with stirring; l) separately ingredient 18 was poured with stirring into purified water in a dissolver equipped with a turbine homogeniser, m) when dissolution of ingredient 18 was complete, ingredients 15, 16 and 17 were added with stirring. Stirring was then continued for 15 minutes; n) the solution from step k) was mixed with the dispersion from step m) and purified water. The thus obtained dispersion was filtered on a mesh having square openings of 0.0225 mm² (N.B.: stirring of this gastroresistant coating dispersion was maintained throughout the film-coating operation), o) the coated cores from step h) were coated with the gastroresistant dispersion from step n) by spraying in a coating pad according to the following parameters: tablet temperature: 45 - 49°C, bowl rotation: 8 r.p.m., spray pressure: 1.2 bar, total spraying time: 4 hours, mean weight of final tablets: 1485 mg, | | |

Dissolution tests performed on the gastroresistant tablets so obtained showed that they did not release the active ingredient at either pH 1 or pH 6, but did release it progressively at pH 7.2.

The repeatability of the abovementioned method was checked by producing two lots of 15,000 tablets each on a pilot scale.

## Claims

1. A gastroresistant tablet having (i) a core comprising butyric acid, or a salt or an ester thereof as an active ingredient, and at least one pharmaceutically acceptable excipient, (ii) an intermediate coating, and (iii) an outer gastroresistant coating, **characterized in that** the said intermediate coating comprises a cellulose compound and the said gastroresistant coating remains unchanged at an acidic pH and dissolves at pH ≥ 7.2.

2. A gastroresistant tablet according to Claim 1, **characterized in that** the said active ingredient is sodium butyrate.

3. A gastroresistant tablet according to Claim 1 or 2, **characterized in that** the said cellulose compound is selected from the group comprising hydroxypropylmethyl cellulose, methyl cellulose, ethylmethyl cellulose, hydroxymethyl cellulose, and mixtures thereof.

4. A gastroresistant tablet according to Claim 3, **characterized in that** the said intermediate coating further comprises at least one ingredient selected from the group comprising microcrystalline cellulose, stearic acid, colloidal anhydrous silica, talc and PEG.

5. A gastroresistant table according to any one of preceding Claims 1 to 4, **characterized in that** the said gastroresistant coating comprises shellac.

6. A gastroresistant tablet according to claim 5, **characterized in that** the said gastroresistant coating further comprises at least one ingredient selected from the group comprising triethyl citrate, ammonium carbonate, talc, titanium dioxide, yellow iron oxide and vanillin.
